# EUROPÄISCHE PATENTSCHRIFT

(11) **EP 1 353 900 B1**
(45) Veröffentlichungstag und Bekanntmachung des Hinweises auf die Patenterteilung: **04.10.2006**
(21) Anmeldenummer: 02706594.5
(22) Anmeldetag: 08.01.2002
(51) Int. Cl.: C07C 247/12

(54) **5-AZIDOLÄVULINSÄURE, VERFAHREN ZU DEREN HERSTELLUNG SOWIE DEREN VERWENDUNG**
5-AZIDO-LAEVULINIC ACID, METHOD FOR THE PRODUCTION THEREOF AND ITS USE
ACIDE 5-AZIDOLEVULINIQUE, PROCEDE PERMETTANT DE LE PRODUIRE ET SON UTILISATION

(30) Priorität: 08.01.2001 DE 10101317
(43) Veröffentlichungstag der Anmeldung: 22.10.2003
(73) Patentinhaber: Aldenkortt, Sven, 97076 Würzburg (DE)
(72) Erfinder: Aldenkortt, Sven, 97076 Würzburg (DE)
(74) Vertreter: Schubert, Klemens
(86) Internationale Anmeldenummer: PCT/DE2002/000074
(87) Internationale Veröffentlichungsnummer: WO 2002/053532

(56) Entgegenhaltungen:
- EP-A- 0 845 457
- US-A- 5 907 058
- HA H-J ET AL: "SELECTIVE BROMINATION OF KETONES. A CONVENIENT SYNTHESIS OF 5-AMINOLEVULINIC ACID" SYNTHETIC COMMUNICATIONS, MARCEL DEKKER, INC., BASEL, CH, Bd. 24, Nr. 18, 1994, Seiten 2557-2562, XP008008563 ISSN: 0039-7911 in der Anmeldung erwähnt

## Beschreibung

Die vorliegende Erfindung betrifft 5-Azidolävulinsäure, ein Verfahren zu deren Herstellung sowie deren Verwendung zur Herstellung von 5 Aminolävulinsäurehydrochlorid.

Die Bedeutung von 5-Aminolävulinsäurehydrochlorid als Edukt für die Synthese verschiedenster Verbindungen sowie seine vielfältigen Einsatzbereiche, wie beispielsweise in der Medizin, wird nachfolgend erläutert.

5-Aminolävulinsäure, lagerfähig in Form ihres Halogensäureadditionssalzes, insbesondere 5-Aminolävulinsäurehydrochlorid, ist eine bioorganische Verbindung, die als Vorstufe auf dem Tetrapyrrolbiosyntheseweg von Porphobillinogen, Chlorophyll, Häm, Vitamin B12 und Cytochrom auftritt (K. D. Gibson et al., Biochem. J. 1955, 61, 618; S. I. Beale, Plant Physiol. 1971, 48, 316; C. A. Rebeiz, Plant Physiol. 1970, 46, 543; A. I. Scott, Angew. Chem. 1993, 105, 1281-1302; Angew. *Chem.* Int. Ed. Engl. 1993, 32, 1223).

Es ist bekannt, dass diese Verbindung oder ihre Alkylester als Edukte für die Synthese einer Vielzahl von Verbindungen eingesetzt wird.

5-Aminolävulinsäurehydrochlorid findet Anwendung als Substrat für den Assay der 5-Aminolävulisäure-Dehydratase (D. Shemin et al., Methods Enzymol. 1970, 17(A), 205; D. L. Coleman, ibid. 211; A. M. del C. Beatle et al., ibid., 216; P. Bodlaender et al., Anal. Biochem. 1974, 58, 500; D. Gurne, D. Shemin, Methods Enzymol. 1976, 44, 844), wird in der Agrochemie als selektives Herbizid (5-Aminolävulinsäurehydrochlorid: C. A. Rebeiz et al., Enzyme Microb. Technol. 1984, 6, 390; Chem. & Eng. News 1984, 62, 8; S. O. Duke, C. A. Rebeiz, Porphyric Pesticides: Chemistry, Toxikology, and Pharmaceutical Applications. ACS Symposium Series 559 1994; Alkylester von 5-Aminolävulinsäurehydrochlorid: H. Takeya, Jpn. Kokai Tokkyo Koho JP 0409360, 1992; Chem. Abstract. 1992, 116, 19755) und Insektizid (C. A. Rebeiz et al., Pestic. Biochem. Physiol. 1988, 30, 11; A. I. Scott, Angew. Chem. 1993, 105, 1281-1302; Angew. Chem. Int. Ed. Engl. 1993, 32, 1223; S. O. Duke, C. A. Rebeiz, Porphyric Pesticides: Chemistry, Toxikology, and Pharmaceutical Applications. ACS Symposium Series 559 1994) eingesetzt und findet in verschiedenen Bereichen der Medizin Anwendung.

Zu den medizinischen Einsatzbereichen von 5-Aminolävulinsäurehydrochlorid gehört insbesondere die Onkologie, wo es u. a. bei der Bekämpfung von Actiner Keratosis mit Hilfe der photodynamischen Therapie (PDT) (Drugs Fut. 2000, 25(1), 74-76 und darin zitierte Literatur) genutzt wird.

Aber auch in der photodynamischen Diagnostik (PDD), hierzu gehören u. a. einige Anwendungen der PDT, z. B. die Fluoreszenzdetektion einiger Krebsarten wie Lungen-, Blasen- und Prostatakrebs (WO-A 93/20810, WO-A 96/39188, WO-A 98/09155; A. G. Hofstetter, Springer Verlag, Berlin, Heidelberg, New York, London, Paris, Tokio, Hong Kong, Barcelona, Budapest, 1995.), sowie in der Gehirntumorenmarkierung (W. Stummer et al., Neurosurgery 1998, 42(3), 518-526.) greift man auf 5-Aminolävulinsäurehydrochlorid zurück.

Eine neuartige Anwendung betrifft die Photosensibilisierung von Arterien in Kombination mit der Ballon-Anglioplastie, die eine operative Nachbehandlung bei Arterienverkalkung überflüssig machen soll (New Scientist 1999, 162, Nr. 2185).

Auch über eine Anwendung von 5-Aminolävulinsäurehydrochlorid als Haarwuchsmittel wurde kürzlich berichtet (Shiseido Co., Ltd.; Cosmo Sogo Kenkyusho K. K., Jpn. Kokai Tokkyo Koho JP 11116,446 [99116, 446], 1999; Chem. Abstract. 1999, 130, 356898z).

Das große Interesse an 5-Aminolävulinsäurehydrochlorid spiegelt sich auch an der Vielzahl der in der Literatur beschriebenen Synthesewege wieder.

Die Diskussion der verschiedenen Verfahren muss jedoch im Hinblick auf ihre Übertragbarkeit auf die technische Produktion erfolgen, damit diese in so vielen Bereichen einsetzbare Verbindung in großem Maßstab kostengünstig und ohne große Umweltbelastung hergestellt werden kann.

Einige beispielhafte Herstellungsverfahren von 5-Aminolävulinsäurehydrochlorid, ausgehend von verschiedenen Substanzklassen, werden im Folgenden diskutiert.

US-A 5,380,935 offenbart ein Verfahren, in dem Furfuralamin, dessen Aminofunktion geschützt wurde, mit Sauerstoff photochemisch in Gegenwart eines Sensibilisators oxidiert wird, die erhaltene Zwischenverbindung katalytisch reduziert und das entstandene Zwischenprodukt unter Abspaltung der Schutzgruppe im Sauren zu 5-Aminolävulinsäurehydrochlorid hydrolysiert wird.

Auch einige andere Verfahren machen Gebrauch von Furanderivaten als Ausgangsmaterial zur Synthese von 5-Aminolävulinsäurehydrochlorid (EP-A 0607952; US-A 5,284,973; US-A 5,344,974; L. Cottier, G. Descotes, L. Eymard, K. Rapp, Synthesis 1995, 303-306; K. Suzuki et al., Nippon Kagaku Kaishi 1999, 3, 199-202).
Diesen Verfahren ist gemeinsam, dass eine Aminogruppe geschützt und die Schutzgruppe im letzten Teilschritt der Synthesesequenz wieder abgespalten werden muss. Es werden z. T. für Anwender und Umwelt bedenkliche Lösungsmittel und Chemikalien verwendet, die nach Abschluß der Reaktion kostenintensiv entsorgt werden müssen.

Auch die Verwendung von teuren Photosensibilisatoren wie Fulleren (C60) oder Rose Bengal sprechen gegen eine technische Anwendung der Verfahren.

Alternativ werden Succinsäurederivate unter Einführung einer Kohlenstoff-Stickstoffeinheit für die Synthese von 5-Aminolävulinsäurehydrochlorid eingesetzt.

In US-A 3,846,490 wird beispielsweise Succinsäuremonochloridmonomethylester mit Hippursäure in γ-Picolin als Lösungsmittel zur Reaktion gebracht. Das erhaltene Oxazolinderivat wird unter Bildung von 5-Aminolävulinsäurehydrochlorid hydrolysiert.

Weitere Verfahren, die von Succinsäure ausgehen, werden von A. Pfaltz, Tetrahedron Lett. 1984, 25, 2977-2980 und A. Nudelman, A. Nudelman, Synthesis 1999, 5, 568-570 beschrieben.

Leider sind in diesen Reaktionssequenzen die einzelnen Aufarbeitungsschritte aufwendig und führen z. T. zu Nebenprodukten, die abgetrennt werden müssen.

Für die Herstellung von 5-Aminolävulinsäurehydrochlorid in den hier beschriebenen Verfahren werden toxische Chemikalien (z. B. Zink, Kupfercyanid) und Lösungsmittel (z. B. γ-Picolin) verwendet. Die in Teilschritten der Synthese entstehenden toxischen Nebenprodukte müssen entsorgt werden.

Pyridinderivate finden als Ausgangsmaterial für die Synthese von 5-Aminolävulinsäurehydrochlorid ebenfalls Anwendung.

So erhielten C. Herdeis et al. in Arch. Pharm. 1984, 317, 304-306 aus 2,5-Dihydroxypyridin durch Oxidation ein Zwischenprodukt, das nach katalytischer Hydrierung 2,5-Piperidindion ergab. Saure Hydrolyse führte zu 5-Aminolävulinsäurehydrochlorid.

H. Takeya, K. Suzuki, K. Sasaki, Nippon Kagaku Kaishi 1999, 355-358 verwenden als Edukt 1,5-Dihydroxy-2-pyridon zur Herstellung von 5-Aminolävulinsäurehydrochlorid.

Bei diesen Reaktionssequenzen handelt es sich um Synthesen, die sowohl Oxidations- als auch Reduktionsschritte enthalten. Die in beiden Verfahren erhaltenen schlechten Ausbeuten an 5-Aminolävulinsäurehydrochlorid stehen in einem ungünstigen Verhältnis zum Aufwand der Verfahren. Außerdem gestaltet sich die Bereitstellung großer Mengen der benötigten Pyridinderivate schwierig.

Die im Folgenden aufgeführten Verfahren zur Herstellung von 5-Aminolävulinsäurehydrochlorid gehen von kostengünstiger und in großer Menge erhältlicher Lävulinsäure aus (Produktion im Tonnenmaßstab: J. J. Bozell, L. Moens, D. C. Elliott, Y. Wang, G. G. Neuenschwander, S. W. Fitzpatrick, R. J. Bilski, J. L. Jarnefeld, Res. Cons. Recyc. 2000, 28, 227-239).

In US-A 5,907,058 wird 5-Bromlävulinsäuremethylester, den man durch Bromierung von Lävulinsäure in Methanol erhält (S.F. McDonald, Can. J. Chem. 1974, 52, 3257-3258), mit Natriumdiformylamid zum 5-Diformylaminolävulinsäuremethylester umgesetzt. Anschließende saure Hydrolyse ergibt 5-Aminolävulinsäurehydrochlorid.

Alternative Verfahren, bei denen von 5-Bromlävulinsäuremethylester ausgegangen und Brom durch ein Stickstoffnucleophil an der C-5-Position nucleophil substituiert wird, wurden beschrieben (Kaliumphthalimid als Stickstoffquelle: E. Benedikt, H.-P. Köst, Z. Naturforsch. 1986, 41b, 1593-1594; Umsetzung mit Natriumazid: H.-J. Ha, S.-K. Lee, Y.-J. Ha, J.W. Park, Synth. Comm. 1994, 24(18), 2557-2562).

Im Falle der Substitution durch ein Azid erhält man den 5-Azidolävulinsäureester, der in Form eines schweren Öls erhalten wird.

Für die weitere Umsetzung zu 5-Aminolävulinsäurehydrochlorid durch katalytische Hydrierung und gleichzeitige Esterhydrolyse, muss sichergestellt sein, dass der Azidoester rein vorliegt.

Der Azidoester lässt sich jedoch nicht ohne Zersetzung destillieren und andere Reinigungsschritte, wie beispielsweise chromatographische Verfahren sind sehr aufwendig und damit für den technischen Maßstab nicht zu empfehlen.

Ein weiterer großer Nachteil der zuletzt beschriebenen Synthesesequenzen liegt in der schwierigen Handhabbarkeit von 5-Bromlävulinsäuremethylester.

Diese Verbindung liegt in flüssiger Form vor, besitzt stark tränen- und hautreizende Eigenschaften und neigt in Gegenwart von Säurespuren (z. B. Bromwasserstoff) zur säurekatalysierten Isomerisierung in die Verbindungen 3-Brom-, 3,5-Dibrom- und Lävulinsäuremethylester, wodurch die Lagerung der Verbindung erschwert wird.

Die Substitution von Brom im 5-Bromlävulinsäuremethylester durch ein Alkalimetallimid oder -azid liefert als Nebenprodukt Natriumbromid, dessen mäßig bis gute Lösungseigenschaften in organischen Lösungsmitteln, insbesondere niederen Alkoholen bekannt sind.

Die Herstellung von Natriumbromidfreiem 5-Aminolävulinsäurehydrochlorid erfordert deshalb z. T. aufwendige Reinigungsschritte.

Insbesondere im Hinblick auf die Verwendung von 5-Aminolävulinsäurehydrochlorid für medizinische Zwecke muss jedoch sichergestellt sein, dass der Wirkstoff sehr rein vorliegt und möglichst keine Verunreinigungen in Form anorganischer Salze enthält.

Anzustreben ist es, dass man einen Reaktionsweg wählt, bei dem man ausgehend von einem reinen Ausgangsprodukt durch eine einfache Transformation quantitativ ein ebenso reines Reaktionsprodukt erhält. Es sollten keine Nebenreaktionen eintreten, wodurch Nebenprodukte entstünden, die wiederum vom erwünschten Hauptprodukt abgetrennt werden müssten.

Im Vergleich zu Verfahren, die nicht von Lävulinsäure ausgehen, bietet die Funktionalisierung durch Brom an der C-5-Position von Lävulinsäure den Vorteil, dass man ausgehend von reinem 5-Bromlävulinsäuremethylester in zwei Stufen problemlos zur 5-Aminolävulinsäurehydrochlorid gelangt und bei Verwendung chemisch reiner Reagenzien keine organischen Nebenprodukte entstehen, die den weiteren Reaktionsweg beeinflussen und aufwendig abgetrennt werden müssen.

Als Nebenprodukte entstehen lediglich Natriumbromid und im Falle der Hydrolyse von Phthalimidolävulinsäuremethylester und Diformylaminolävulinsäuremethylester Phthalsäure bzw. Ameisensäure und Methanol als Hydrolyseprodukte, deren Entsorgung keine Probleme bereitet.

Eine Reaktionssequenz, die von den reinen Lävulinsäurederivaten 5-Bromlävulinsäure und insbesondere 5-Chlorlävulinsäure ausgeht, diese Carbonsäuren im nächsten Schritt durch Substitution des Halogenatoms mit einem anorganischen Azid in die 5-Azidolävulinsäure überführt wird und die Azidofunktion der so erhaltenen 5-Azidolävulinsäure zur Aminofunktion reduziert wird, ist bisher nicht bekannt.

Die Carbonsäuren 5-Brom- und 5-Chlorlävulinsäure sind auf klassischem Weg durch Bromierung und Chlorierung von Lävulinsäure aufgrund der mangelnden Regioselektivität nur schwer zu erhalten. Man erhält die gewünschten Lävulinsäurederivate nur in sehr schlechter Ausbeute.

Beide Verbindungen haben jedoch die vorteilhafte Eigenschaft, dass sie in kristalliner Form vorliegen, keine tränenreizenden Eigenschaften besitzen und lagerfähig sind.

Besonders vorteilhaft ist die Umsetzung von 5-Chlorlävulinsäure mit Natriumazid, da man als Nebenprodukt lediglich unschädliches Natriumchlorid erzeugt.

Aufgabe der vorliegenden Erfindung ist es deshalb eine Verbindung und ein Verfahren zur Herstellung dieser Substanz bereitzustellen, wobei die Verwendung dieser Verbindung zur Herstellung von 5-Aminolävulinsäurehydrochlorid die Nachteile des Standes der Technik überwindet.

Die Aufgabe wird durch die Bereitstellung von 5-Azidolävulinsäure gelöst.

5-Azidolävulinsäure ist eine bisher nicht bekannte Verbindung und ein neues Edukt zur Herstellung von 5-Aminolävulinsäurehydrochlorid.

Ein weiterer Gegenstand der vorliegenden Erfindung ist ein Verfahren zur Herstellung von 5-Azidolävulinsäure.

Weiterhin ist ein Gegenstand der vorliegenden Erfindung die Verwendung von 5-Azidolävulinsäure als Edukt für eine in technischem Maßstab geführte, kostengünstig und in hoher Ausbeute verlaufenden Synthese zur Herstellung von 5-Aminolävulinsäurehydrochlorid in pharmazeutischer Qualität.

Durch die Bereitstellung von 5-Azidolävulinsäure ist es möglich, ein verbessertes Herstellungsverfahren von 5-Aminolävulinsäurehydrochlorid zu schaffen. Das Verfahren ermöglicht es, 5-Aminolävulinsäurehydrochlorid in technischem Maßstab kostengünstig, in hoher Ausbeute und in pharmazeutischer Reinheit herzustellen.

Erfindungsgemäß wird die Aufgabe durch ein Verfahren gelöst, das es ermöglicht, 5-Aminolävulinsäurehydrochlorid in einem 4-Stufen-Prozeß mit einer Gesamtausbeute von 31-35 % in pharmazeutischer Reinheit darzustellen.

Bei dem erfindungsgemäßen Verfahren werden kostengünstige Edukte verwendet und als zu entsorgende Nebenprodukte lediglich Bromwasserstoff, Methanol und Natriumchlorid erzeugt.

Überraschenderweise wurde ein einfacher Weg gefunden 5-Chlorlävulinsäure aus 5-Bromlävulinsäuremethylester herzustellen.

5-Bromlävulinsäuremethylester, den man auf bekannte Weise durch Bromierung von Lävulinsäure in Methanol erhält, reagiert mit wässriger Salzsäure unter Esterhydrolyse und gleichzeitigem Brom/Chlor-Austausch an der C-5-Position quantitativ zu 5-Chlorlävulinsäure. Diese Transformation ist bisher unbekannt.

Die Verbindung stellt einen hervorragend kristallisierenden Feststoff dar (Schmp. 75°C), der keine tränenreizenden Eigenschaften besitzt und unbegrenzt lagerfähig ist.

Die Verbindung ist für die Umsetzung mit einem Stickstoffnucleophil wie Natriumazid besser geeignet als das Bromderivat, da als einziges Nebenprodukt Natriumchlorid entsteht. Natriumchlorid ist in organischen Lösungsmitteln praktisch unlöslich und nicht toxisch.

Die so erhaltene 5-Chlorlävulinsäure wird im Folgeschritt in einem geeigneten Lösungsmittel gelöst und mit der equivalenten Menge eines geeigneten Azids, insbesondere Natriumazid, gerührt.

Die Reaktion wird vorzugsweise in Aceton durchgeführt. Aber auch andere organische Lösungsmittel wie dipolar aprotische Lösungsmittel, substituierte und unsubstituierte Amide, cyclische und acyclische Ether können verwendet werden.

Ein Arbeiten mit anderen Lösungsmitteln, die ein vergleichbares Lösungsverhalten zeigen, insbesondere Lösungsmittel hoher Polarität, soll nicht ausgeschlossen sein.

Als neue Schlüsselverbindung entsteht durch nucleophile Substitution quantitativ die noch nicht bekannte Verbindung 5-Azidolävulinsäure.

Diese neue Schlüsselverbindung 5-Azidolävulinsäure stellt, wenn sie nach dem nachfolgend beschriebenen Verfahren hergestellt wird, eine farblose, kristalline Verbindung dar (Schmp. 70-71°C) und ist unbegrenzt und ohne Zersetzung lagerfähig. Die Substanz enthält kein anorganisches Material, ist schlagunempfindlich und kann bei Raumtemperatur sicher gehandhabt werden.

Aufgrund eines unvollständigen Brom/Chlor-Austauschs kann die 5-Chlorlävulinsäure bis zu 8 % 5-Bromlävulinsäure enthalten. Das Vorhandensein von geringen Mengen 5-Bromlävulinsäure stört den weiteren Reaktionsverlauf jedoch nicht, da auch diese Verbindung mit einem geeigneten anorganischen Azid, insbesondere Natriumazid, in Aceton glatt unter Bildung von 5-Azidolävulinsäure reagiert.

Die Umsetzung von auf anderem Wege hergestellter 5-Bromlävulinsäure mit Natriumazid in Aceton ergab nach Kristallisation aus einem organischen Lösungsmittel beinahe quantitativ reine 5-Azidolävulinsäure. Die Verbindung war frei von anorganischen Salzen.

Im Folgeschritt wird die Schlüsselverbindung 5-Azidolävulinsäure problemlos zu 5-Aminolävulinsäurehydrochlorid reduziert. Vorzugsweise führt man die Reduktion als katalytische Hydrierung in Anwesenheit eines Metallkatalysators (vorzugsweise Palladium oder Platin auf einem geeigneten Träger wie Aktivkohle) in wässriger Salzsäure durch. Der Katalysator kann regeneriert werden. Es entstehen keine unerwünschten organischen Nebenprodukte. Man erhält 5-Aminolävulinsäurehydrochlorid in absolut reiner Form in einer Gesamtausbeute von 31-35 % (ausgehend von Lävulinsäure).

Erfindungsgemäß wird die Aufgabe also dadurch gelöst, dass 5-Azidolävulinsäure zur Verfügung gestellt wird und zu deren Herstellung ein Verfahren bereitgestellt wird, bei dem man zur Herstellung von 5-Azidolävulinsäure 5-Bromlävulinsäuremethylester und/oder 5-Chlorlävulinsäuremethylester mit wässriger Salzsäure umsetzt und infolge eines unvollständigen Brom/Chloraustauschs an der C-5-Position ein Gemisch aus 5-Chlor- und 5-Bromlävulinsäure erhält, dann die erhaltene 5-Chlorlävulinsäure, ein Gemisch aus 5-Chlor- und 5-Bromlävulinsäure und die reine 5-Bromlävulinsäure durch Umsetzung mit einem geeigneten nucleophilen Azid in 5-Azidolävulinsäure überführt.

Erfindungsgemäß bevorzugt ist es, dass man die Umsetzung mit dem Azid in einem polaren Lösungsmittel, insbesondere Aceton, einem C1-C4-Alkanol oder Wasser, als Reaktionsmedium durchführt.

Besonders bevorzugt ist es, dass man als Azid ein Alkaliazid, insbesondere Natriumazid, verwendet.

Erfindungsgemäß wird die Aufgabe weiterhin gelöst, indem 5-Azidolävulinsäure zur Herstellung von 5-Aminolävulinsäurehydrochlorid verwendet wird, wobei man 5-Azidolävulinsäure durch katalytische Hydrierung in 5-Aminolävulinsäurehydrochlorid überführt.

Insbesondere bevorzugt ist es, dass man die katalytische Hydrierung in wässriger Salzsäure durchführt, den verwendeten Hydrierkatalysator abtrennt, das Lösungsmittel und überschüssige Salzsäure abdestilliert und man 5-Aminolävulinsäurehydrochlorid durch Auskristallisieren aus 2-Propanol oder t-Butylmethylether/Methanol erhält.

Die folgenden Beispiele erläutern die Erfindung.

### Beispiel 1

### Herstellung von 5-Chlorlävulinsäure:

Man rührt eine Lösung von 1 g (4,78 mmol) 5-Bromlävulinsäuremethylester in 20 ml 3 m Salzsäure bei 70°C bis zum Ende der Reaktion (¹H-NMR-Kontrolle, 24 Stunden). Man destilliert das Lösungsmittel, die überschüssige Salzsäure, Bromwasserstoff und Methanol im Vakuum ab, gibt 20 ml Dichlormethan hinzu, trocknet mit Natriumsulfat, destilliert das Lösungsmittel und erhält 706 mg eines blassgelben Feststoffs.

Man nimmt den Feststoff in 2 ml t-Butylmethylether auf und gibt soviel Petroleumbenzin (40-60°C) hinzu, bis Kristallisation einsetzt. Man rührt noch 0,5 Stunden bei 20-25°C und saugt die weißen Kristalle ab.
**Ausbeute**: 684 mg, 95 %.
**Schmelzpunkt**: 75°C; eine Röntgenstrukturanalyse der Kristalle belegen die Struktur der Verbindung.
**Elemtaranalyse:** Ber.: C 39,89; H 4,69. Gef.: C 39,35; H 4.67.
**IR (KBr)**: ν (cm⁻¹) = 3500-2100, 1700 (C=O), 1440, 1410, 1370, 1330, 1290, 1210, 1140, 1090, 1030, 950, 865, 765, 730, 610.
¹H-NMR (CDCl₃, 200 MHz): δ (ppm) = 2,70 (t, ³J = 6.7 Hz, 2 H); 2,96 (t, ³J = 6.7 Hz, 2 H); 4,14 (s, 2 H); 8,8 (br. s, 1 H).
¹³C-NMR (CDCl₃, 50, 3 MHz): δ (ppm) = 27, 7 (HOOC-**C**H2), 34,0 (CH2-**C**H2-C=O); 48,0 (**C**H2-Cl); 178,4 (**C**OOH); 201,2 (**C**=O).

### Beispiel 2

### Herstellung eines Gemisches von 5-Chlorlävulinsäure und 5-Bromlävulinsäure:

Man rührt eine Lösung von 1 g (4,78 mmol) 5-Bromlävulinsäuremethylester in 20 ml 3 m Salzsäure 12 Stunden bei 70°C. Man destilliert das Lösungsmittel, überschüssige Salzsäure, Bromwasserstoff und Methanol im Vakuum, gibt 20 ml Dichlormethan hinzu, trocknet mit Natriumsulfat, destilliert das Lösungsmittel und erhält 740 mg eines blaßgelben Feststoffs.

Man nimmt den Feststoff in 2 ml t-Butylmethylether auf und gibt so viel Petroleumbenzin (40-60°C) hinzu, bis Kristallisation einsetzt. Man rührt noch 0,5 Stunden bei 20-25°C und saugt die weißen Kristalle ab.
^{**1**}**H-NMR :**

Nach Integration der 5-CH2-Protonensignale ergibt sich für das Gemisch aus 5-Chlorlävulinsäure und 5-Bromlävulinsäure ein Verhältnis von 92 : 8.
**Ausbeute:** 700 mg, 95 %.

### Beispiel 3

### Herstellung von 5-Azidolävulinsäure aus 5-Chlorlävulinsäure:

Die gemäß Beispiel 1 erhaltene 5-Chlorlävulinsäure wird in Aceton gelöst und nach Zugabe von Natriumazid im Verhältnis 1:1 bei 50°C bis zum Ende der Reaktion gerührt
(¹H-NMR-Kontrolle).
Man gibt 20 ml Dichlormethan hinzu, filtriert den Feststoff, wäscht mit halbgesättigter, wäßriger Natriumchloridlösung, trocknet mit Natriumsulfat, destilliert das Lösungsmittel und erhält quantitativ ein blaßgelbes Öl.

Aus Dichlormethan/Petroleumbenzin (40-60°C) kristallisiert die 5-Azidolävulinsäure in Form blaßgelber Nadeln.
**Ausbeute**: 700 mg, 98 %.
**Schmelzpunkt**: 70-71°C.
**Elementaranalyse**: Ber.: C 38;22; H 4,49; N 26,74. Gef.: C 38,39; H 4,59; N 26,07.
**IR** (KBr): ν (cm⁻¹) = 3350-2350, 2090 (N3), 1725 (C=O), 1415, 1400, 1370, 1340, 1285, 1260, 1230, 1170, 1080, 1040, 1000, 930, 885, 830, 800, 685, 630.
^{**1**}**H-NMR** (CDCl₃, 200 MHz): δ (ppm) = 2,70 (s, 4 H); 4,02 (s, 2 H); 8,3 (br. s, 1 H).
^{**13**}**C-NMR** (CDCl₃, 50,3 MHz): δ (ppm) = 27,4 (HOOC-CH₂); 34,1 (CH₂-**C**H₂-C=O); 57,5 (**C**H₂-N₃); 177,3 (**C**OOH); 202,7 (**C**=O).

### Beispiel 4

### Herstellung von 5-Azidolävulinsäure aus einem Gemisch von 5-Chlor- und 5-Bromlävulinsäure:

0,50 g des gemäß Beispiel 2 erhaltenen Gemischs aus 5-Chlor- und 5-Bromlävulinsäure werden in Aceton gelöst und nach Zugabe von Natriumazid im Verhältnis 1:1 bei 50°C bis zum Ende der Reaktion gerührt (¹H-NMR.Kontrolle). Man gibt 20 ml Dichlormethan hinzu, filtriert den Feststoff, mit Natriumsulfat, destilliert das Lösungsmittel und erhält quantitativ ein gelbes Öl.
Aus Dichlormethan/Petroleumbenzin (40-60°C) kristallisiert die 5-Azidolävulinsäure in Form blaßgelber Nadeln.
**Ausbeute**: 510 mg, 98 %;

### Physikalische und spektroskopische Daten:

Die physikalischen und spektroskopischen Daten stimmten mit den Daten der erhaltenen Verbindung aus Beispiel 3 überein.

### Beispiel 5

### Herstellung von 5-Azidolävulinsäure aus 5-Bromlävulinsäure:

0,50 g (2,56 mmol) 5-Bromlävulinsäure werden in 10 ml Aceton gelöst und nach Zugabe von Natriumazid im Verhältnis 1:1 bei 20-25°C bis zum Ende der Reaktion (¹H-NMR-Kontrolle) gerührt.

Man gibt 20 ml Dichlormethan hinzu, filtriert den Feststoff, wäscht mit halbgesättigter, wäßriger Natriumchloridlösung, trocknet mit Natriumsulfat, destilliert das Lösungsmittel und erhält quantitativ ein orangerotes Öl.

Aus Dichlormethan/Petroleumbenzin kristallisiert die 5-Azidolävulinsäure in Form blaßgelber Nadeln.
**Ausbeute**: 390 mg, 97 %;

### Physikalische und spektroskopische Daten:

Die physikalischen und spektroskopischen Daten stimmten mit den Daten der erhaltenen Verbindung aus Beispiel 3 überein.

### Beispiel 6

### Hydrierung von 5-Azidolävulinsäure zu 5-Aminolävulinsäurehydrochlorid:

Die gemäß Beispiel 3 erhaltene 5-Azidolävulinsäure wird in halbkonzentrierter Salzsäure gelöst, mit einem Hydrierungskatalysator (Palladium auf Kohle) versetzt, und unter Einleiten von Wasserstoff mit einem Druck von 6 bar wird die eingesetzte Verbindung 5 Stunden lang hydriert. Durch eine Überwachung der Hydrierung zeigt sich, daß die Hydrierung nach 5 Stunden quantitativ abgelaufen ist (¹H- und ¹³C-NMR-Kontrollspektren zeigten nur noch Produktsignale).

Der Katalysator wird daraufhin von der klaren, farblosen Reaktionslösung filtriert, und das Reaktionsmedium wird im Vakuum entfernt.

Es wird ein klebriger, farbloser Rückstand erhalten, der unter Rühren mit 4 ml 2-Propanol versetzt wird. Alternativ löst man den Rückstand in 5 ml Methanol auf und gibt 5 ml t-Butylmethylether zu. Nach kurzer Zeit bilden sich farblose Kristalle, die über eine Glasfritte abgesaugt werden.

Die Kristalle werden mit Aceton gewaschen und im Vakuum getrocknet.
**Ausbeute**: 709 mg, 95 %
**Schmelzpunkt**: 150-151°C (Zersetzung).
**Schmelzpunktangaben verschiedener Hersteller: Sigma:** 144-151°C; Merck: 150-156°C; Fluka: ~ 150°C (Zersetzung); Aldrich: 156°C (Zersetzung); Acros Organics 156-158°C (Zersetzung).

Die NMR-spektroskopischen (A. Nudelman and A. Nudelman, Synthesis 1999, 4, 568-570) und chromatographischen Daten (C. Herdeis, A. Dimmerling, Arch. Pharm. 1984, 317, 304-306) stimmen mit den in der Literatur angegebenen Daten überein.

### Beispiel 7

### Herstellung von 5-Bromlävulinsäure:

In einem Dreihalskolben mit Tropftrichter, Innenthermometer und Rückflußkühler tropft man unter Rühren zu einer Lösung von 5,0 g (43,1 mmol) Lävulinsäure in 50 ml Tetrachlormethan bei 20-25°C innerhalb von 0,5 Stunden 6,88 g (43, 1 mmol) Brom.

Nach Entfärbung der anfangs orangeroten Lösung gibt man 50 ml Wasser hinzu, trennt die organische Phase ab, wäscht mit gesättigter, wäßriger Natriumchloridlösung, trocknet mit Magnesiumsulfat, destilliert das Lösungsmittel und erhält ein gelbes Öl.

Aus 50 ml Diethylether/Petroleumbenzin (40-60°C) kristallisieren farblose Kristalle, die aus reiner 5-Bromlävulinsäure bestehen.
**Ausbeute**: 900 mg, 9.5 %.
**Schmelzpunkt**: 79-80°C.
**IR** (KBr): ν (cm⁻¹) = 3500-2100, 1700 (C=O), 1430, 1400, 1350, 1300, 1280, 1240, 1100, 1050, 970, 920, 860, 760, 720, 610.
^{**1**}**H-NMR** (CDCl₃, 200 MHz): δ (ppm) = 2,70 (t, ³J = 6.7 Hz, 2 H); 2,96 (t, ³J = 6.7 Hz, 2 H); 3,95 (s, 2 H); 8,5 (br. s, 1 H).
^{**13**}**C-NMR** (CDCl₃, 50,3 MHz): δ (ppm) = 28,0 (HOOC-**C**H2); 34,1 (CH2-**C**H2-C=O) ; 33,9 (CH2-Br); 178,0 (**C**OOH); 200, 4 (**C**=O).

## Patentansprüche

1. 5-Azidolävulinsäure.

2. Verfahren zur Herstellung von 5-Azidolävulinsäure, wobei man
a) 5-Bromlävulinsäuremethylester und/oder 5-Chlorlävulinsäuremethylester mit wäßriger Salzsäure umsetzt und infolge eines unvollständigen Brom/Chloraustausch an der C-5-Position ein Gemisch aus 5-Chlor- und 5-Bromlävulinsäure erhält, und
b) die erhaltene 5-Chlorlävulinsäure, ein Gemisch aus 5-Chlor- und 5-Bromlävulinsäure und die reine 5-Bromlävulinsäure durch Umsetzung mit einem nucleophilen Azid in 5-Azidolävulinsäure überführt.

3. Verfahren nach Anspruch 2, **dadurch gekennzeichnet, daß** man die Umsetzung mit dem Azid in einem polaren Lösungsmittel, insbesondere Aceton, einem C₁-C₄₋Alkanol oder Wasser, als Reaktionsmedium durchführt.

4. Verfahren nach Anspruch 2, **dadurch gekennzeichnet, daß** man als Azid ein Alkaliazid, insbesondere Natriumazid, verwendet.

5. Verwendung von 5-Azidolävulinsäure zur Herstellung von 5-Aminolävulinsäurehydrochlorid, wobei man 5-Azidolävulinsäure durch katalytische Hydrierung in 5-Aminolävulinsäurehydrochlorid überführt.

6. Verwendung nach Anspruch 5, **dadurch gekennzeichnet, daß** man
a) die katalytische Hydrierung in wäßriger Salzsäure durchführt,
b) den verwendeten Hydrierkatalysator abtrennt,
c) das Lösungsmittel und überschüssige Salzsäure abtrennt und man
d) 5-Aminolävulinsäurehydrochlorid durch Auskristallisieren aus 2-Propanol oder t-Buthymethylether/Methanol erhält.

## Claims

1. 5-Azido levulinic acid

2. Process for the preparation of 5-azidolevuliniv acid, wherein
(a) methyl 5-bromo levulinate and/or methyl 5-chloro levulinate is converted with aqueous hydrochloric acid and as a result of an incomplete bromine/chlorine exchange at the C-5-postion a mixture of 5-chloro levulinic acid and 5-bromo levulinic acid is obtained, and
(b) the obtained 5-chloro levulinic acid, a mixture of 5-chloro levulinic acid and 5-bromo levulinic acid and the pure 5-bromo levulinic acid is transferred into 5-azido levulinic acid by conversion with a nucleophilic azide.

3. Process according to Claim 2, wherein in stage (b) the conversion with an azide is carried out in a polar solvent, especially acetone, a C₁-C₄-alkanol or water as reaction medium.

4. Process according to Claim 2, wherein in stage (b) an alkali metal azide, in particular sodium azide is used as nucleophilic azide.

5. Use of 5-azido levulinic acid for the preparation of 5-amino levulinic acid hydrochloride, whereas 5-azido levulinic acid is converted into 5-amino levulinic acid hydrochloride by catalytic hydrogenation.

6. Use according to Claim 5, wherein (a) the catalytic hydrogenation ist carried out in aqueous hydrochloric acid, (b) the solvent and excess hydrochloric acid is seperated and (c) 5-amino levulinic acid hydrochloride is obtained by cristallisation from 2-propanol or *t-butyl* methyl ether/methanol.

## Revendications

1. Acide 5-azidolévulinique

2. Procédé de préparation de l'acide 5-azidolévulinique, dans lequel
a) on laisse réagir bromo-5-lévulinate de méthyle et/ou chloro-5-lévulinate de méthyle avec l'acide chlorhydrique aqueuse et, à cause d'échange brome/chlore incomplet, on obtient un mélange de l'acide 5-chlorolévulinique et de l'acide 5-bromolévulinique, et
b) on transforme l'acide 5-chlorolévulinique obtenue, le mélange de l'acide 5-chloro- et 5-bromolévulinique et l'acide 5-bromolévulinique en l'acide 5-azidolévulinique par réaction avec un azoture nucléophil.

3. Procédé selon la revendication 2, dans lequel on effectue la transformation avec l'azoture dans un solvant polaire, en particulier acétone, C₁ - C₄₋alcanol ou eau.

4. Procédé selon la revendication 2, dans lequel on utilise comme l'azoture un azoture de métal alcalin, en particulier l'azoture de sodium.

5. Utilisation de l'acide 5-azidolévulinique pour la préparation de l'acide 5-aminolévulinique chlorhydrate, dans laquelle on transforme l'acide 5-azidolévulinique en l'acide 5-aminolévulinique chlorhydrate par hydrogénation catalytique.

6. Utilisation selon la revendication 5, dans laquelle
a) on effectue l'hydrogénation catalytique dans l'acide chlorhydrique aqueuse,
b) on sépare le catalysateur utilisé,
c) on sépare le solvant et l'acide chlorhydrique excédentaire et
d) on obtient l'acide 5-aminolévulinique chlorhydrate par cristallisation dans le propane-2-ol ou dans l'oxyde de tert-butyle et méthyle/méthanol.
